# EUROPEAN PATENT APPLICATION

(11) **EP 4 312 224 A1**
(43) Date of publication of application: **31.01.2024**
(21) Application number: 22187118.9
(22) Date of filing: 27.07.2022
(51) Int. Cl.: G16H 30/40, G16H 50/20

(54) **A PATIENT-SPECIFIC ARTIFICIAL NEURAL NETWORK TRAINING SYSTEM AND METHOD**

(71) Applicant: Siemens Healthcare GmbH, 91052 Erlangen (DE)
(72) Inventor: Engel, Thomas, 73432 Aalen (DE); Brock, Jens-Peter, 90579 Langenzenn (DE); Marquardt, Gaby, 91353 Hausen (DE)
(74) Representative: Siemens Healthineers Patent Attorneys

(57) **Abstract**

The invention relates to a training system and a training method for training an artificial neural network, in particular for medical applications, with patient-specific features. The patient-specific artificial neural network training system comprises an input interface, configured to receive image data from a patient, a computing device, further comprising a classification module, a separator module and a training module, and an output interface, configured to output a diagnosis signal, wherein the classification module is configured to acquire as input the received image data and generate a first classification signal for each image of the image data, wherein the separator module is configured to, based on the first classification signal, separate the received image data in at least a first dataset and a second dataset according to a reliability criterion, and wherein the training module is configured to use the first dataset or only a part thereof as a training dataset to train an artificial neural network.

## Description

The present invention relates to a training system and method to train an artificial neural network, preferably for medical applications, taking into account patient-specific features. In particular, the invention relates to improving the performance of artificial neural networks as classifiers and tools for assisted diagnosis and making them more adaptable to the characteristics of each patient.

The invention will, by way of example, be mostly applied to the fields of hematology and immunology, and in particular to the classification of white blood cells, but the principles of the invention have a broader scope.

Medical diagnosis is a field where the introduction of computer-aided applications and, in particular, artificial intelligence and machine learning, has opened a vast array of possibilities. In particular, computerized clinical decision support systems have been evolving continuously since their introduction forty years ago. In the last years, some countries have moved to the HIMSS Stage 6 and the use of electronic health records (EHR) is the rule rather than the exception.

Artificial neural networks offer one of the most promising ways to assist in medical decision taking. In samples where a large volume of training data is available, artificial intelligence entities are competitive with the decisions made by unassisted humans.

More problematic are those cases where data is scarce. Likewise, for rare cases only sparsely covered by the training data, the number of outliers can be too small for the neural network to identify them and extract meaningful information. In the fields of medicine and biology this can happen for markers of rare diseases, but also in the diagnosis of a particular patient. Data of a particular patient is scarce compared to the average data gathered from the population of patients. Deviations from the average are to be expected, and in some cases it may hinder the diagnosis. For example, a patient might show low levels of iron in blood or low blood pressure without them being indicative of a disease. In the classification of white blood cells, a patient could show, for example, abnormal basophiles with granulation below the average, again more as a feature rather than a marker of a disease.

Before diagnosing a patient, a human expert would try to gather as much information about the patient as possible, in order to, to the extent possible, adapt to patient-specific characteristics and reach a correct diagnosis. It is challenging to incorporate this patient-specific knowledge into artificial neural networks, but at the same time it is an important issue in order to improve computerized assisted-diagnosis systems.

It is an object of the present invention to provide a training system and method to train an artificial neural network to be used as a tool for assisted diagnosis in medical applications, with an emphasis in adapting the artificial neural network to the characteristics of a patient in order to improve the diagnostic accuracy.

The purpose of this invention will be achieved through a device with the features disclosed in claim 1 and a method with the features detailed in claim 14. Preferred embodiments of the invention with advantageous features are given in the dependent claims.

A first aspect of the invention provides a patient-specific artificial neural network training system, comprising an input interface, configured to receive image data from a patient; a computing device, further comprising a classification module, a separator module and a training module; and an output interface, configured to output a diagnosis signal; wherein the classification module is configured to acquire as input the received image data and generate a first classification signal for each image of the acquired image data, wherein the separator module is configured to, based on the first classification signal, separate the received image data in at least a first dataset and a second dataset according to a first reliability criterion, and wherein the training module is configured to use the first dataset or only a part thereof as a training dataset to train an artificial neural network.

The input and/or the output interfaces are broadly understood as entities capable of acquiring image data and delivering it for further processing. Each of them may therefore contain, at least, a central processing unit, CPU, and/or at least one graphics processing unit, GPU, and/or at least one field-programmable gate array, FPGA, and/or at least one application-specific integrated circuit, ASIC and/or any combination of the foregoing. Each of them may further comprise a working memory operatively connected to the at least one CPU and/or a non-transitory memory operatively connected to the at least one CPU and/or the working memory. Each of them may be implemented partially and/or completely in a local apparatus and/or partially and/or completely in a remote system such as by a cloud computing platform.

The input interface and/or the output interface may be realized in hardware and/or software, cable-bound and/or wireless, and in any combination thereof. Any of the interfaces may comprise an interface to an intranet or the Internet, to a cloud computing service, to a remote server and/or the like.

Image data broadly describes any visual information that can be used for further processing and analysis in medical applications. In the context of blood cell classification, image data might consist of images of artificially-stained blood samples (or: stained blood smears), using any of the staining methods used in blood cell classification. Some of these image data might comprise a selected sub-image portion or a crop of a larger image, where segmentation has been applied and/or the crop contains objects to be classified together with some surrounding background. In the following, image data, image dataset or images will indistinctly be used to describe information in image form.

Likewise, training dataset herein refers mostly to training images. Both terms will be used henceforth without distinction in meaning.

Whenever herein an artificial neural network is mentioned, it shall be understood as a computerized entity able to implement different data analysis methods broadly described under the terms artificial intelligence, machine learning, deep learning or computer learning. The artificial neural network can be a generative adversarial network (GAN), a convolutional neural network (CNN) or any other neural network. The artificial neural network to be trained will also be referred to as a refined classifier or a patient-specific artificial neural network.

The computing device is to be broadly understood as an entity able to process data. The computing device can be realized as any device containing or consisting of, at least, a central processing unit, CPU, and/or at least one graphics processing unit, GPU, and/or at least one field-programmable gate array, FPGA, and/or at least one application-specific integrated circuit, ASIC and/or any combination of the foregoing. It may further comprise a working memory operatively connected to the at least one CPU and/or a non-transitory memory operatively connected to the at least one CPU and/or the working memory. The computing device may execute a software, an app or an algorithm with different capabilities for data processing. It may be implemented partially and/or completely in a local apparatus and/or partially and/or completely in a remote system such as by a cloud computing platform.

A diagnosis signal may comprise an indication that a specific disease or condition may be or even is present in a patient. A diagnosis signal may comprise a list of possible or probable diseases associated with the classified images, once they have been analysed by the patient-specific artificial neural network.

A second aspect of the present invention provides a computer-implemented patient-specific training method, preferably to be used with the patient-specific training system of the first aspect, comprising the following steps: (a) acquiring image data from a patient; (b) generating a first classification signal for each image of the acquired image data; (c) separating the image data into at least a first dataset and a second dataset based on the first classification signal according to a first reliability criterion; (d) initializing an artificial neural network; and (e) training the artificial neural network with the entire first dataset or a part thereof.

In particular, the method according to the second aspect of the invention may be carried out with the image-forming device according to the first aspect of the invention. The features and advantages disclosed herein in connection with the image-forming device are therefore also disclosed for the method, and vice versa.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention.

One of the main ideas underlying the present invention is to provide a training system, configured to train an artificial intelligence neural network for the classification of medical image data, which is able to capture patient-specific features. This is achieved by processing a set of images from a patient through a classification module (or pre-trained classifier) and, based on the output, split the images into two groups. A first group comprises the images that have been identified with high confidence and a second group the images that could not be identified with good enough confidence. Out of the images of the first group a training image dataset is generated, with which a patient-specific artificial neural network is trained. This patient-specific artificial neural network is a refined classifier, which contains more information about the patient than the pre-trained classifier.

The device as described above affords a simple implementation of a method to train an artificial neural network for the classification of medical images, which can be used as a subsequent diagnosis-assistance tool. One step consists in acquiring the medical images, which in most cases have previously undergone a staining process. In another step each image acquires a classification signal, which e.g. indicates the likelihood that an image belongs to the different classes of the classifier. Based on this classification signal and a quantitative labelling criterion, the images can be separated into the first and the second dataset. An (untrained) artificial neural network is initialized and trained with part of the first dataset, which contain the images which could be identified with higher certainty.

One advantage of the present invention is that by doing a refined classification through the training of an artificial neural network with the most indicative cells, patient-specific features are acquired and learned by the artificial neural network. The classification accuracy and the diagnostic accuracy of the patient-specific artificial neural network are therefore higher than those that can be achieved with an artificial neural network that has been trained with data from many patients. This process of acquiring patient-specific features mimics how humans would incorporate patient-specific characteristics into their diagnoses.

Another advantage of the present invention is that the performance of the patient-specific artificial neural network can be easily tested by inputting the images of the second dataset in the already trained artificial neural network. In this test run the quality of the refined classifier can be evaluated by comparing the classification signal of the refined classifier with the one achieved with the pre-trained classifier. If the training has been successful, the reliability measure of the images of the second dataset should increase and in some cases one can even identify and label all or part of the images of the second dataset with confidence.

Advantageous embodiments and further developments follow from the dependent claims as well as from the description of the different preferred embodiments illustrated in the accompanying figures.

According to some of the embodiments, refinements, or variants of embodiments, the classification module comprises a pre-trained artificial neural network structure.

According to some of the embodiments, refinements, or variants of embodiments, the classification signal indicates, for each image, probability values associated to a first set of classes. In other words, the artificial neural network can post-process the probability distribution associated to an image and assign a class label to the image.

According to some of the embodiments, refinements, or variants of embodiments, the first dataset comprises all images above a threshold probability value and the second dataset comprises all images at or below the same threshold probability value. The first dataset contains the very indicative cells of the different classes of the first set of classes. Introducing an absolute probability threshold is a simple way of separating this first dataset from the less conclusive cases that will define the second dataset. It is also possible, and in some applications advantageous, to split the images in more than two datasets by e.g. setting different probability thresholds.

According to some of the embodiments, refinements, or variants of embodiments, the first dataset comprises all images above a relative threshold probability value between the classes and the second dataset comprises all images at or below the same relative threshold probability value between the classes. In certain cases, it is advantageous to define the reliability of the classification based on a relative probability measure. This reliability criterion emphasizes the distinguishability of one class with respect to the others. According to some of the embodiments, refinements, or variants of embodiments, the training module further comprises a selector unit, which is configured to determine a second set of classes and assemble the training dataset, based on information from the first dataset and/or the second dataset. While the first set of classes is determined by the pre-trained artificial neural network of the classification unit, the second set of classes can be determined prior to the training of the artificial neural network. This depends on the outcome of the classification performed by the classification unit. If, for instance, the representatives of the first dataset only populate significantly a subset of classes, it might be preferable to train the artificial neural network with only those well-represented classes. On the other hand, the second dataset might show that the unclear cases mostly arise from the confusion between two classes. In this case, it might be advantageous to concentrate the refined classification on these two classes, since this is where the classification can most likely be improved. The selection of the specific training dataset, e.g. the proportion of images from each of the selected classes, is also a parameter that can be adjusted depending on the class-distribution of the first dataset. In certain preferred embodiments, the second set of classes might be a subset of the first set of classes. In certain other embodiments, the second set of classes might contain classes that were not present in the first set of classes.

According to some of the embodiments, refinements, or variants of embodiments, the selector unit further comprises a threshold discriminating subunit, which is configured to select a class of the first set of classes as a class of the second set of classes, provided that the number of images classified in the class of the first set of classes is above a certain threshold value. In other words, the configuration of the second set of classes can take place based on a quantitative criterion, which only selects those classes that are statistically significant by defining a lower threshold of representatives per class.

According to some of the embodiments, refinements, or variants of embodiments, the selector unit further comprises a sampling subunit, which is configured to sample a subset of data from the first dataset and/or augment the first dataset, such that each class of the second set of classes has a statistically significant number of images in the training dataset. Even with the introduction of a threshold, some classes might be underrepresented with respect to other selected classes. This might lead to an unwanted bias in the training of the artificial neural network. In order to avoid this possible bias it is advantageous to balance the amount of representatives per class. In general, the sampling subunit will take a random selection of representatives of the overrepresented classes to have a balanced distribution of the training images according to the second set of classes. The sampling subunit can also apply augmentation techniques to generate further training data for less well-represented or rare classes, e.g. by rotating, mirroring, flipping, scaling and/or resampling image data. In general, increasing the amount of qualified training data by augmentation will lead to a better performance of the artificial neural network.

According to some of the embodiments, refinements, or variants of embodiments, the selector unit further comprises an artificial intelligence subunit pre-trained to recognize a specific diagnosis, which is configured to select the training images based on a second reliability criterion. In some cases where a specific disease or abnormality is the target of the analysis of the patient images, the second set of classes should be related to the markers of the specific disease or abnormality. In this case, using an artificial neural network pre-trained to identify the specific disease or abnormality can select the training dataset more efficiently. Depending on the data available, the artificial neural network can be trained with generic data or patient-specific data. The reliability criterion may be a probability distribution among the different classes with which the artificial neural network has been pre-trained.

According to some of the embodiments, refinements, or variants of embodiments, the training module is further configured to generate a second classification signal associated with each of the images of the training dataset, wherein the second classification signal indicates, for each image of the training dataset, probability values associated to the second set of classes.

According to some of the embodiments, refinements, or variants of embodiments, the selector unit further comprises a training-scenario subunit, configured to select the artificial neural network of the training module. This artificial neural network can be either based on the artificial neural network of the classification module or be based on a different model. The training-scenario subunit is able to process different models, whose performance can be later evaluated and compared.

According to some of the embodiments, refinements, or variants of embodiments, the training-scenario subunit is further configured to add patient-specific non-image data to the training dataset used to train the artificial neural network of the training module. The artificial neural network training dataset is thus enlarged by patient-based metadata.

According to some of the embodiments, refinements, or variants of embodiments, the patient-specific non-image data comprises information on at least a previous diagnosed disease. This existing diagnosis of the patient selects the kind of algorithm to be used, in particular one that is pre-trained to identify the specific diagnosed disease. The artificial neural network could be trained on generic data or data from the patient itself, depending on the data availability. According to some of the embodiments, refinements, or variants of embodiments, the patient-specific training system further comprises a quality-control module, which is configured to input image data of the second dataset into the already-trained artificial neural network of the training module and to compare for each image the second classification signal with the first classification signal. In order to test the quality of the refined classification one can use the artificial neural network of the training module with the second dataset, i.e. the dataset considered non-conclusive in the classification performed by the classification module. The probability distribution obtained by the pre-trained artificial neural network can then be compared with the probability distribution of the patient-specific artificial neural network. A successful training is achieved if images of the second dataset would be better identified, i.e. their probability distribution would now lead to a safe identification or at least to an improvement of the identification.

According to some of the embodiments, refinements, or variants of embodiments, the patient-specific training system further comprises a validation module, which is configured to input image data of the first dataset into the already-trained artificial neural network of the training module and to compare for each image the second classification signal with the first classification signal. A validation step can be defined by inputting images of the first dataset not used for training into the already trained artificial neural network. The refined classifier should correctly label the images, preferably with an increased confidence level.

Although here, in the foregoing and also in the following, some functions are described as being performed by modules, it shall be understood that this does not necessarily mean that such modules are provided as entities separate from one another. In cases where one or more modules are provided as software, the modules may be implemented by program code sections or program code snippets, which may be distinct from one another but which, may also be interwoven or integrated into one another.

Similarly, in cases where one or more modules are provided as hardware, the functions of one or more modules may be provided by one and the same hardware component, or the functions of several modules may be distributed over several hardware components, which need not necessarily correspond to the modules. Alternatively and in addition, one or more of such modules can also be related to a more specific computing structure as it can be realized with FPGA technologies. Thus, any apparatus, system, method and so on which exhibits all of the features and functions ascribed to a specific module shall be understood to comprise, or implement, said module. In particular, it is a possibility that all modules are implemented by program code executed by the computing device, for example a server or a cloud computing platform.

According to some of the embodiments, the method of the second aspect further comprises the following steps: validating the artificial neural network with part of the image data of the first dataset, wherein a second classification signal is generated for each image and compared with the first classification signal of the same image, and evaluating the performance of the artificial neural network with the image data of the second dataset, wherein a second classification signal is generated for each image and compared with the first classification signal of the same image.

According to some of the embodiments, the patient-specific training system is part of the computer-assisted clinical diagnosis embedded inside a diagnostic decision support system (DDSS) to aid the human decision-making in the clinical workflow.

The above embodiments and implementations can be combined with each other as desired, as far as this is reasonable. Further scope of the applicability of the present method and apparatus will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

Aspects of the present disclosure will be better understood with reference to the following figures. The components in the drawings are not necessarily to scale, emphasis being placed instead upon clearly illustrating the principles of the present disclosure. Parts in the different figures that correspond to the same elements have been tagged with the same reference numerals in the figures.
Fig. 1 is a schematic illustration of a patient-specific training system according to an embodiment of the present invention;
Fig. 2 is a schematic depiction of the structure of a selector unit according to some embodiments of the present invention;
Fig. 3 is a block diagram showing an exemplary embodiment of the patient-specific training method;
Fig. 4 is an example of a possible image processing according to some embodiments of the present invention;
Fig. 5 is a schematic block diagram illustrating a computer program product according to an embodiment of the third aspect of the present invention; and
Fig. 6 is a schematic block diagram illustrating a non-transitory computer-readable data storage medium according to an embodiment of the fourth aspect of the present invention.

The figures might not be to scale and certain components can be shown in generalized or schematic form in the interest of clarity and conciseness. In some instances, well-known structures and devices are shown in block diagram form in order to avoid obscuring the concepts of the present invention. Likewise, the numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

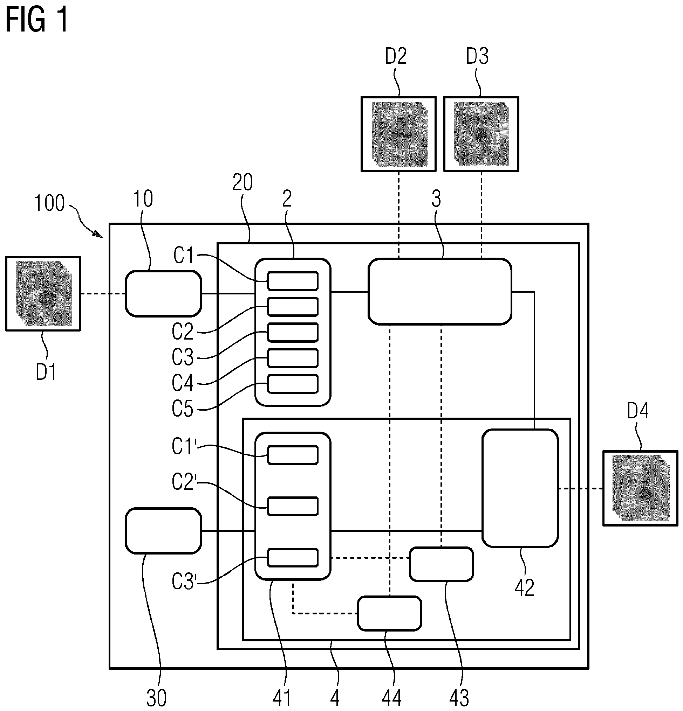

Fig. 1 shows a schematic illustration of a patient-specific training system 100 according to an embodiment of the present invention. For illustrative purposes, the training system of Fig. 1 is designed to train an artificial neural network for the classification of white blood cells. However, it should be understood that this particular example does not limit the scope of the invention.

The training system 100 comprises an input interface 10, a computing device 20 and an output interface 30. The input interface 10 is broadly understood as any entity capable of acquiring image data D1 and delivering it for further processing.

The computing device 20 is to be broadly understood as any entity capable of processing data. The computing device 20 illustrated in Fig. 1 comprises a classification module 2, a separator module 3 and a training module 4. The classification module 2 is configured to acquire as input the image data D1 and classify it according to some classification criteria. In its simplest version, the classification module 2 comprises a pre-trained artificial neural network. In the particular application described in Fig. 1, the artificial neural network has been pre-trained to classify the different types of white blood cells in e.g. basophils C1, monocytes C2, lymphocytes C3, blasts C4 and myelocytes C5. The classification module 2 outputs a first classification signal that ascribes a probability distribution to each of the images D1, which measures the likelihood that an image D1 belongs to each of the classes C1-C5.

The separator module 3 is configured to interpret the probability distribution for each image D1 and separate the images D1 in at least a first dataset D2 and a second dataset D3. The first dataset D2 contains the images D1 that can be ascribed to one of the classes C1-C5 with certainty, while the second dataset D3 contains the images D1 whose identification is deemed as uncertain.

According to some of the embodiments of the invention, the criterion to label an image as certain is determined by a probability threshold. For instance, an image becomes an identification label to a class if the associated probability is above 70%. This probability threshold can be predetermined, e.g. it may be part of an existing classification protocol, or it can be decided a posteriori, based on the obtained probability distribution. With this criterion of an absolute probability threshold, a first image with a probability distribution (75, 5, 2, 10, 8) would be kept in the first dataset D2 and be classified as a basophil (probability of 75%), while a second image with a probability distribution (60, 20, 5, 5, 10) would be kept in the second database D3.

According to some of the embodiments of the invention, a relative probability might, in some cases, be preferred. For instance, an image is labelled as identified with certainty if the probability difference between the highest probability and the following one exceeds 30%. Returning to the previous example, with this criterion based on a relative probability, both the first image, with probability distribution (75, 5, 2, 10, 8), and the second image, with probability distribution (60, 20, 5, 5, 10) would be kept in the first dataset D2. Both images would be classified as a basophil (probability of 75% and 60%, respectively.

The training module 4 is configured to input the first dataset D2 or only a part thereof to train an artificial neural network 41. It comprises or consists of an artificial neural network 41 to be trained, a selector unit 42, a quality-control module 43 and a validation module 44.

The artificial neural network 41 can be e.g. a generative adversarial neural network (GAN) or a convolutional neural network (CNN). Fig. 1 shows, by way of example, an artificial neural network 41 with three classes C1' to C3'. As discussed before, the classes C1'-C3' preferably are a subset of the classes C1-C5 of the classification module 2.

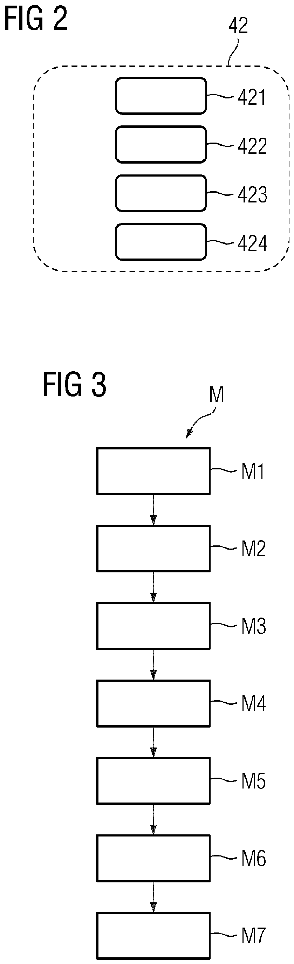

The selector unit 42 is configured to generate the training dataset D4 out of the first dataset D2. In some embodiments it is also configured to choose the artificial neural network 41 to be trained and the classes C1'-C3' for the classification. In order to perform these functions, the selector unit 42 can be endowed with a threshold discriminating subunit 421 and/or a sampling subunit 422 and/or an artificial intelligence subunit 423 and/or a training-scenario subunit 424. The structure of the selector unit 42 is discussed in detail with respect to Fig. 2.

The quality-control module 43 and the validation module 44 can be employed once the artificial neural network 41 has been trained. The quality-control module 43 is configured to input images of the second dataset D3, which has not been used for training, into the already-trained patient-specific artificial neural network 41. In order to assess the performance of the patient-specific artificial neural network 41 compared to the pre-trained classifier of the classification module 2, the quality-control module 43 compares the first classification signal and the second classification signal for each of the images of the second dataset D3. As already discussed above, these classifications signals are reliability measures, and in some embodiments they will be realized as probability distributions over all the available classes of the classifier.

The quality-control module 43 compares these probability distributions. This way it can be quantitatively determined how well the artificial neural network 41 has learned patient-specific features. The probability distributions for the images in the second dataset D3 from the second classification signal should outperform the ones of the first classification signal. Ideally, this improvement is good enough that images of the second dataset D3, which could not be identified with enough confidence by the separator module 3, they can now be reclassified and identified with confidence. This reclassification of the images D1 can also be done by the separator module 3, using the same probability thresholds employed when processing the first classification signal, or modifying them.

The validation module 44 is configured to input images of the first dataset D2, preferably those not employed for the training in order to avoid e.g. overfitting, into the already-trained patient-specific artificial neural network 41. In order to validate the artificial neural network 41, the first classification signal for each of the images is compared to the corresponding second classification signal. Since these images were already classified by the separator module 3 with high confidence based on the first classification signal, it is to be expected that the second classification signal will simply confirm the classification, probably with a higher degree of confidence.

The output interface 30 is broadly understood as any entity capable of generating a diagnosis signal based on the information acquired from the training module 4. It may therefore contain, at least, a central processing unit, CPU, and/or at least one graphics processing unit, GPU, and/or at least one field-programmable gate array, FPGA, and/or at least one application-specific integrated circuit, ASIC and/or any combination of the foregoing. It may further comprise a working memory operatively connected to the at least one CPU and/or a non-transitory memory operatively connected to the at least one CPU and/or the working memory. It may be implemented partially and/or completely in a local apparatus and/or partially and/or completely in a remote system such as by a cloud computing platform.

If the training system 100 is to be implemented as part of a computer-assisted clinical diagnosis embedded inside a diagnostic decision support system (DDSS), the output interface 30 can present the diagnosis signal on a user interface such as a website, an application, a software or an electronic health records (EHR) front-end interface.

Both the input interface 10 and/or the output interface 30 may be realized in hardware and/or software, cable-bound and/or wireless, and in any combination thereof. Any of the interfaces 10, 30 may comprise an interface to an intranet or the Internet, to a cloud computing service, to a remote server and/or the like.

Fig. 2 shows a schematic depiction of the structure of a selector unit 42 according to some embodiments of the present invention. The selector unit 42 is configured to decide, based on information from the first dataset D2 and the second dataset D3, which training dataset D4 out of the first dataset D2 is to be used to train the artificial neural network (41 in Fig. 1). Furthermore, it can also determine the kind of artificial neural network 41 to be used, the training algorithm, as well as the selection of classes (C1'-C3' in Fig. 1) that the artificial neural network 41 will use to classify the images.

Fig. 2 shows a possible configuration of the selector unit 42, comprising a threshold discriminating subunit 421, a sampling subunit 422, an artificial intelligence subunit 423 and a training-scenario subunit 424.

The threshold discriminating subunit 421 is configured to select a certain class of the first set of classes (C1-C5 in Fig. 1) as a class of the second set of classes (C1'-C3' in Fig. 1), provided that the number of images classified in the certain class of the first set of classes C1-C5 is above a certain threshold value. The reduction of data when going from the received images D1 to the first dataset D2 means that it might be statistically meaningful to have the second set of classes C1'-C3' smaller than the first set of classes C1-C5. The threshold value applied by the threshold discrimination subunit 421 is a measure of the statistical significance that is wanted for the training of the classes of the second set of classes C1'-C3'. A less sophisticated option is to simply keep a fixed number of classes (two, three or more) out of the first set of classes C1-C5 when training the artificial neural network 41. These classes are preferably the ones with the highest number of representatives for the training.

The sampling subunit 422 is configured to sample a subset of data from the first dataset D2 and/or augment the first dataset D2, such that each class of the second set of classes C1'-C3' has a statistically significant number of representatives. The sampling subunit 422 can be used in conjunction with the threshold discriminating subunit 421, in order to make sure that there is no imbalance between the selected second set of classes C1'-C3' with which the artificial neural network 41 will be trained. It is important to keep the number of training representatives of each class more or less even, or at the very least avoid certain classes to be underrepresented or overrepresented. The sampling subunit 422 may perform a random selection to crop representatives from the most represented classes, or it can simply chop off the representatives of those classes hierarchically, starting from those representatives with lower identification certainty. Depending on the capability of data, image augmentation might also be used to balance the training dataset D4, with the additional benefit that it increases the overall number of representatives, thereby increasing the expected classification quality of the artificial neural network 41.

The artificial intelligence subunit 423 is configured to perform a selection of the second set of classes C1'-C3' based on a specific diagnosis. The artificial intelligence subunit 423 comprises at least an artificial neural network (not shown in the figure) pre-trained with data in order to recognize a specific disease. Since in some of the embodiments the training system 100 is meant to be part of a computer-assisted diagnostic system, fixing the second set of classes C1'-C3' by using pre-trained artificial neural networks can be certainly advantageous. For example, one might want to use the training system 100 to ascertain whether a patient which was diagnosed in the past with blood cancer suffers it again. The artificial intelligence subunit 423 can be trained with the blood samples of the patient when he/she was first diagnosed, and used this trained artificial neural network to determine the classes C1'-C3' of the artificial neural network 41. The amount of available data for the training is in this case clearly the limiting factor, but one could also complement patient blood samples with blood samples of other patients diagnosed with the same type of blood cancer.

The training-scenario subunit 424 is configured to select the model and algorithm of the artificial neural network 41. Since it is not always clear which artificial neural network 41 will perform better for a give application, one of the advantages that this operation brings is that different artificial neural network structures can be trained. The training-scenario subunit 424 can then be combined with the quality-control module 43 in order to determine which artificial neural network 41 is best suited for the application at hand.

The training-scenario subunit 424 is further configured to add patient-specific non-image data to the data used to train the artificial neural network of the training module 4. Using meta-data in some artificial neural networks 41 might definitely improve their performance, for instance if the metadata contains information about previous conditions or diseases of a patient.

All these subunits depicted in Fig. 2 can operate in conjunction as long as the respective operations do not lead to contradictions or conflicts.

Fig. 3 is a block diagram showing an exemplary embodiment of the training method M, to be applied preferably with the training system 100 described in Fig. 1. One step M1 comprises acquiring an image dataset D1 of a patient, which in the preferred embodiment of white blood cell classification it has in most cases previously undergone a staining and segmentation process. In another step M2, a first classification signal is generated for each of the acquired images D1. In most of the preferred embodiments, this is achieved by inputting the images D1 in a pre-trained artificial neural network, which outputs a probability distribution for each image as a first classification signal. In another step M3, based on this first classification signal, the image dataset D1 is split in at least a first dataset D2 and a second dataset D3. This separation is performed with some quantitative criteria. How this may be done has been discussed above in the description of Fig. 1. In another step M4 an artificial neural network 41 is initialized and then trained in another step M5, using as training dataset D4 the first dataset D2 or part thereof. How subsets of the first dataset D2 can be defined has been discussed in detail e.g. in the description of Fig. 1 and Fig. 2.

Once the artificial neural network 41 has been trained, it can be validated in another step M6. In this step M6, part of the images of the first dataset D1 not used in the training dataset D4 is to be employed. A second classification signal is generated for each image and compared with the first classification signal of the same image. In most preferred embodiments, this amounts to a comparison of two probability distributions. In another step M7, the performance of the artificial neural network 41 is evaluated by using the images of the second dataset D3. A second classification signal is generated for each image and compared with the first classification signal of the same image. In most preferred embodiments, this amounts to a comparison of two probability distributions.

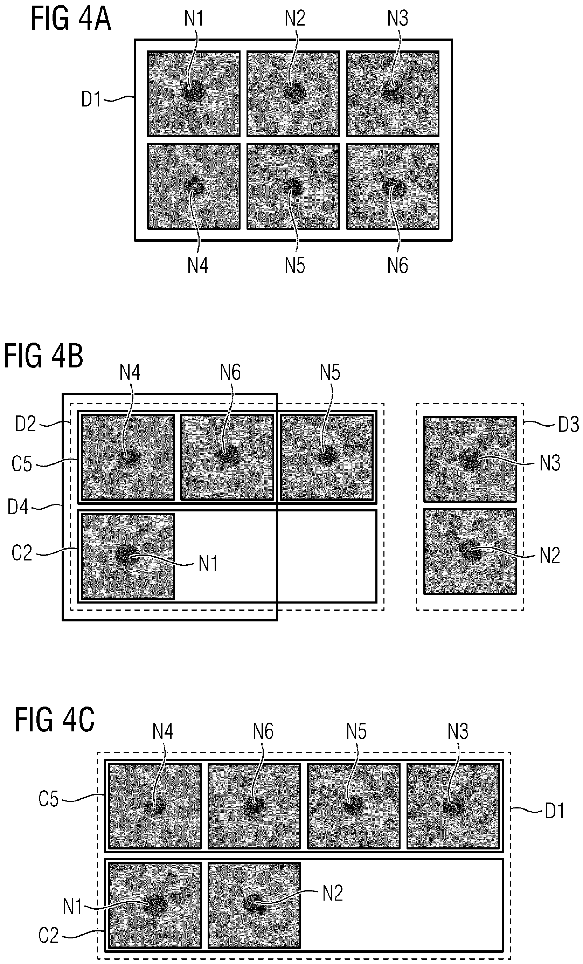

Fig. 4 shows an example of a possible image processing according to some embodiments of the present invention. Fig. 4A shows six color-stained images N1-N6 as an example of the image data D1 received by the input interface 10. They show a normal monocyte N1, a dysplastic monocyte N2, a dysplastic metamyelocyte N3, a normal segmented neutrophil N4, and two dysplastic myelocytes N5, N6. The cells are from a patient with myelodysplastic syndrome (MDS), which is a type of blood cancer where myelocytes, which are precursors of neutrophils, do not mature correctly.

The images N1-N6 are processed by the classification module 2, which, preferably by using a pre-trained artificial neural network, generates a first classification signal, preferably in the form of a probability distribution for each of the images N1-N6 according to a set of classes (C1-C5 in Fig. 1). For simplicity, one can restrict the classes to two types of white blood cells, e.g. monocytes C2 and myelocytes C5. In this case, where the purpose is to determine whether the patient suffers MDS or not, the pre-trained artificial neural network is preferably pre-trained with samples diagnosed with MDS.

Fig. 4B shows the splitting of the images done by the separator module 3. The probability distributions for each of the images N1-N6 lead to labels once a reliability criterion is applied. As discussed before, according to some preferred embodiments, a good criterion is to apply a probability threshold. In the example of Fig. 4B, after this threshold is applied the images N4, N5 and N6 are characterized as myelocytes C5, while the image N1 is identified as a monocyte C2. The first dataset D2 therefore comprises the images N1, N4, N5 and N6. In contrast, the images N2 and N3 are inconclusive and belong to the second dataset D3.

According to the principles of the present invention, this classification can be refined by using the images N1, N4, N5 and N6 of the first dataset D2 or a part thereof, to train an artificial neural network 41 (see Fig. 1). Fig. 4B shows an example of a training dataset D4, which comprises N1, N4 and N6. This could be the result of applying the threshold discriminating subunit 421 or the sampling subunit 422 of the selector unit 42. In the first case, N5 would be under a certain probability threshold. In other words, N5 would be the less certain of the images inside the first dataset D2 and would be left out of the training dataset D4. In the second case, the sampling subunit 422 would be used to reduce the proportion of myelocytes vs monocytes, which are in a 3:1 proportion, to a 2:1 proportion. Leaving N5 out could then be a random process or N5 could be specifically chosen, e.g. because it has the lowest certainty among the myelocytes.

Fig. 4C shows a stage where the artificial neural network 41 has already been trained with the training dataset D4 shown in Fig. 4B. Fig. 4C is an illustration of what one would expect from a successful refined classification when the images N2 and N3 of the second dataset D3 shown in Fig. 4B are inputted in the artificial neural network 41. This operation is managed by the quality-control module 43, which generates a second classification signal, preferably in the form of a probability distribution for the images N2 and N3, which indicates how probable the images N2 and N3 are myelocytes C5 or monocytes C2. We have already discussed above that the separator unit 3 determines whether the images can be identified (i.e. they are determined with certainty, according to the chosen reliability criterion). In the example of Fig. 4C N3 happens to be identified as a myelocyte C5, while N2 is labelled as a monocyte N2.

The image N2 could not be identified with the pre-trained artificial neural network, which has been trained to identify generic monocytes. Instead, the refined training adds patient-specific features that are learned by the artificial neural network 41. The result is that, when the white blood cells of the patient are used for training, N2 is found to be similar to the monocytes of the patient and can be correctly labelled. The final result is a trained artificial neural network 41 that is patient-customized and therefore can provide more reliable information for an assisted diagnosis of, in this particular example, MDS.

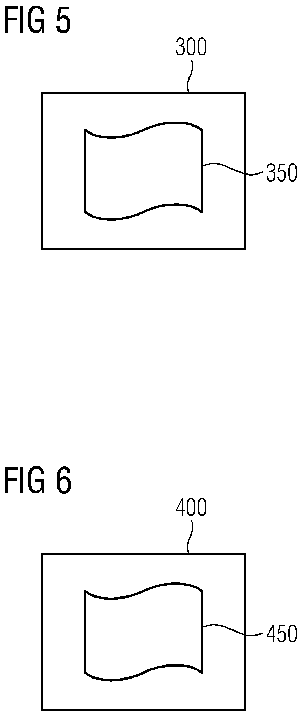

Fig. 5 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as it has been described with respect to the preceding figures.

Fig. 6 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as it has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

### List of Reference Signs

- 2: classification module
- 3: separator module
- 4: training module
- 10: input interface
- 20: computing device
- 30: output interface
- 41: artificial neural network
- 42: selector unit
- 43: quality-control module
- 44: validation module
- 100: patient-specific artificial neural network training system
- 300: computer program product
- 350: executable program code
- 400: non-transitory computer-readable data storage medium
- 421: threshold discriminating subunit
- 422: sampling subunit
- 423: artificial intelligence subunit
- 424: training-scenario subunit
- 450: executable program code
- D1: image data
- D2: first dataset
- D3: second dataset
- D4: training dataset
- N1: normal monocyte
- N2: dysplastic monocyte
- N3: dysplastic metamyelocyte
- N4: normal segmented neutrophil
- N5: dysplastic myelocyte
- N6: dysplastic myelocyte
- C1-C5: first set of classes
- C1'-C3': second set of classes
- M: computer-implemented patient-specific training method
- M1-M7: method steps

## Claims

1. A patient-specific artificial neural network training system (100), comprising:
an input interface (10), configured to receive image data (D1) from a patient;
a computing device (20), further comprising a classification module (2), a separator module (3) and a training module (4); and
an output interface (30), configured to output a diagnosis signal;
wherein the classification module (2) is configured to acquire as input the received image data (D1) and generate a first classification signal for each image of the image data (D1),
wherein the separator module (3) is configured to, based on the first classification signal, separate the received image data (D1) in at least a first dataset (D2) and a second dataset (D3) according to a first reliability criterion, and
wherein the training module (4) is configured to use the first dataset (D2) or only a part thereof as a training dataset (D4) to train an artificial neural network (41).

2. The patient-specific training system (100) according to claim 1, wherein the classification module (2) comprises a pre-trained artificial neural network structure.

3. The patient-specific training system (100) according to claims 1 to 2, wherein the first classification signal indicates, for each image, probability values associated to a first set of classes (C1-C5).

4. The patient-specific training system (100) according to claims 1 to 3, wherein the first dataset (D2) comprises all images above a threshold probability value and the second dataset (D3) comprises all images at or below the same threshold probability value.

5. The patient-specific training system (100) according to claims 1 to 3, wherein the first dataset (D2) comprises all images above a relative threshold probability value between the classes (C1-C5) and the second dataset (D3) comprises all images at or below the same relative threshold probability value between the classes (C1-C5).

6. The patient-specific training system (100) according to claims 1 to 5, wherein the training module (4) further comprises a selector unit (42), which is configured to determine a second set of classes (C1'-C3') and assemble the training dataset (D4) based on information from the first dataset (D2) and/or the second dataset (D3).

7. The patient-specific training system (100) according to any of the previous claims, wherein the selector unit (42) further comprises a threshold discriminating subunit (421), which is configured to select a class of the first set of classes (C1-C5) as a class of the second set of classes (C1'-C3'), provided that the number of images (D1) classified in the class of the first set of classes (C1-C5) is above a certain threshold value.

8. The patient-specific training system (100) according to any of the previous claims, wherein the selector unit (42) further comprises a sampling subunit (422), which is configured to sample a subset of data from the first dataset (D2) and/or augment the first dataset (D2), such that each class of the second set of classes (C1'-C3') has a statistically significant number of images in the training dataset (D4).

9. The patient-specific training system (100) according to any of the previous claims, wherein the selector unit (42) further comprises an artificial intelligence subunit (423), pre-trained to recognize a specific diagnosis, which is configured to select the training dataset (D4) based on a second reliability criterion.

10. The patient-specific training system (100) according to any of the previous claims, wherein the training module (4) is further configured to generate a second classification signal associated with each image of the training dataset (D4), wherein the second classification signal indicates, for each image of the training dataset (D4), probability values associated to the second set of classes (C1'-C3').

11. The patient-specific training system (100) according to any of the previous claims, wherein the selector unit (42) further comprises a training-scenario subunit (424), which is configured to select the artificial neural network (41) of the training module (4).

12. The patient-specific training system (100) according to claim 11, wherein the training-scenario subunit (424) is further configured to add patient-specific non-image data to the training dataset (D4) used to train the artificial neural network (41) of the training module (4).

13. The patient-specific training system (100) according to claim 12, where the patient-specific non-image data comprises information of at least a previous diagnosed disease.

14. A computer-implemented patient-specific training method (M), preferably to be used with the patient-specific training system (100) according to the previous claims, comprising the following steps:
acquiring (M1) image data (D1) from a patient;
generating (M2) a first classification signal for each image of the acquired image data (D1);
separating (M3) the image data (D1) into at least a first dataset (D2) and a second dataset (D3) based on the first classification signal and according to a first reliability criterion;
initializing (M4) an artificial neural network (41); and
training (M5) the artificial neural network (41) with the entire first dataset (D2) or a part thereof.

15. A diagnostic decision support system comprising the patient-specific training system (100) of claims 1 to 13.
